# FASCICULE DE BREVET EUROPEEN

(11) **EP 1 613 256 B1**
(45) Date de publication et mention de la délivrance du brevet: **06.06.2007**
(21) Numéro de dépôt: 04742454.4
(22) Date de dépôt: 07.04.2004
(51) Int. Cl.: A61F 13/08, A41B 11/00, D04B 9/52, D04B 1/26

(54) **ARTICLE OU SOUS-ARTICLE DE CONTENTION**
KOMPRESSIONSKLEIDUNGSSTÜCK
RETENTION OR COMPRESSION GARMENT OR UNDERGARMENT

(30) Priorité: 11.04.2003 FR 0304565
(43) Date de publication de la demande: 11.01.2006
(73) Titulaire: GSL Holding (Société Anonyme), 68300 Saint-Louis (FR)
(72) Inventeur: GANZONI, Stefan, F-4103 Bottmingen (CH); LUN, Bertrand, F-42100 Saint-Etienne (FR)
(74) Mandataire: Nuss, Pierre
(86) Numéro de dépôt international: PCT/FR2004/000865
(87) Numéro de publication internationale: WO 2004/092611

(56) Documents cités:
- WO-A-01/00118
- US-A- 4 172 456
- US-A- 4 571 960
- US-A- 5 005 567
- US-A- 5 412 957
- US-B1- 6 523 729

## Description

La présente invention concerne des traitements de la pathologie veineuse, plus précisément ceux mettant en oeuvre des articles dits de contention ou de compression, et plus particulièrement le domaine de la fabrication d'articles dérivés de véritables articles de contention ou de compression proprement dits, notamment la fabrication de « sous-articles », tels que des sous-bas pour articles de contention.

Par article de contention au sens de la présente description on entend toutes les orthèses compressives tubulaires réalisées en un matériau textile élastique tricoté, à l'exception des bandages, et plus particulièrement celles compressives d'un ou de deux membres inférieurs. Les « sous-articles » de contention au sens de la présente invention sont des articles (typiquement des chaussettes, bas, mi-bas, collants et analogues) destinés à être enfilés en premier et donc à être en contact direct avec la peau du patient. De tels articles sont tout d'abord destinés à améliorer le confort cutané du porteur mais peuvent également avantageusement présenter différentes propriétés visant, par exemple à apporter une légère contention ou à renforcer l'action de contention de l'article principal. En tout état de cause, l'innovation principale des articles précités réside dans le fait qu'ils permettent d'apporter à l'utilisateur à la fois une facilité d'enfilage et un confort augmentés.

En effet, la mise en place sur les membres à traiter, d'articles de contention de classe élevée, typiquement de classe 3 (compression forte comprise entre 27 et 48 hPa) ou de classe 4 (compression extra-forte supérieure à 48 hPa) constitue, encore de nos jours, un réel problème quotidien auquel sont confrontés les patients et/ou le personnel soignant.

Ce problème est d'autant plus critique qu'une mise en place correcte et régulière est indispensable afin d'optimiser l'observance et donc les chances de succès du traitement prescrit par le médecin.

Les difficultés liées à la mise en place sont propres au matériau ou à la matière textile de contention élevée et peuvent même, le cas échéant, être renforcées par un état physiquement diminué du patient, en particulier lorsque ce dernier doit appliquer lui-même ledit traitement.

On connaît déjà de nombreux types de dispositifs d'enfilage mécaniques, en particulier sous la forme d'accessoires généralement pour des bas, qui sont pour la plupart des structures métalliques onéreuses et de manipulation complexe. Ces dispositifs ne sont toutefois pas satisfaisants, les efforts résiduels à fournir par le patient et la complexité de leur utilisation étant encore trop importants et donc dissuasifs.

Il existe donc un réel besoin pour un article simple et économique facilitant de manière significative la mise en place des articles de contention susvisés.

Il existe par ailleurs, des articles de contention qui peuvent être utilisés seuls (pour une contention légère) ou en association avec un article de contention de classe élevée pour en outre renforcer des propriétés déjà existantes de l'article de contention principal. Cependant, l'opération d'enfilage d'un article de contention classique par-dessus un autre présente encore de nos jours des difficultés notoires.

La présente invention a notamment pour objet de pallier les inconvénients précités et de fournir de tels articles.

A cet effet, elle a pour objet un article ou sous-article de contention, pouvant être utilisé seul ou préférentiellement sous un article de contention en étant en contact direct avec la peau du porteur, caractérisé en ce qu'il est essentiellement tricoté avec au moins deux structures de maillage différentes, à savoir :
- une première structure pour une première zone dudit article ou sous-article qui s'étend de l'extrémité prévue pour les orteils jusqu'au niveau des malléoles dudit porteur, cette zone étant tricotée selon un motif dit de « maille flottée », ce dans une matière textile présentant une surface extérieure opposée à celle en contact avec la peau dudit porteur qui soit lisse,
- une seconde structure pour une seconde zone dudit article ou sous-article qui s'étend des malléoles précitées jusqu'à l'autre extrémité opposée à celle prévue pour lesdits orteils, cette zone étant tricotée selon un motif dit de « maille tramée », ce dans une matière textile élastique adaptée présentant un titre de trame compris entre 240 dtex et 600 dtex, de façon à procurer une légère compression ou contention au niveau de ladite seconde zone.

Il peut s'agir pour un tel « sous-article », d'un article placé, par exemple, sous un bas au sens strict (couvrant la cuisse et le jarret), sous un collant (couvrant les deux membres inférieurs et l'abdomen jusqu'à la ceinture, en une seule pièce), sous un mono-collant (collant muni d'une seule jambe, destiné à la contention d'un seul des membres inférieurs) ou encore sous une chaussette (couvrant le jarret seul).

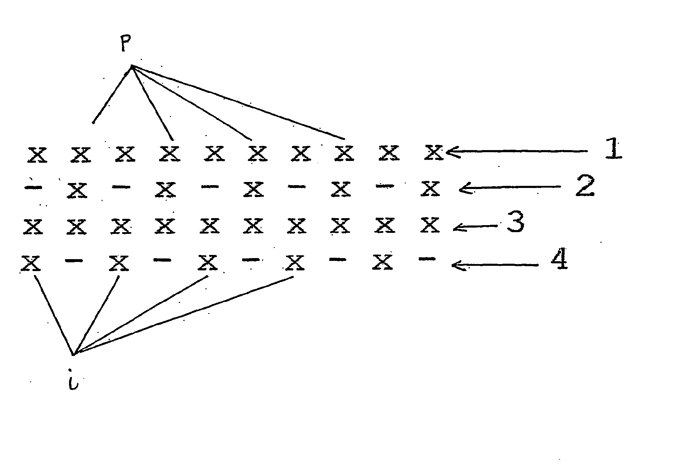

L'invention sera mieux comprise, grâce à la description ci-après, qui se rapporte à des modes de réalisation préférés, donnés à titre d'exemples non limitatifs, et expliqués avec référence au dessin schématique annexé, dans lequel la figure unique est une représentation standardisée de la maille flottée utilisée pour la fabrication d'un article ou sous-article de contention selon l'invention.

Conformément à la présente invention, l'article ou sous-article de contention pouvant être utilisé seul ou préférentiellement sous un article de contention en étant en contact direct avec la peau du porteur est caractérisé en ce qu'il est essentiellement tricoté avec au moins deux structures de maillage différentes, à savoir :
- une première structure pour une première zone dudit sous-article qui s'étend de l'extrémité prévue pour les orteils jusqu'au niveau des malléoles dudit porteur, cette zone étant tricotée selon un motif dit de «maille flottée », ce dans une matière textile présentant une surface extérieure opposée à celle en contact avec la peau dudit porteur qui soit lisse,
- une seconde structure pour une seconde zone dudit sous-article qui s'étend des malléoles précitées jusqu'à l'autre extrémité opposée à celle prévue pour lesdits orteils, cette zone étant tricotée selon un motif dit de « maille tramée », ce dans une matière textile élastique adaptée présentant un titre de trame compris entre 240 dtex et 600 dtex, de façon à procurer une légère compression ou contention au niveau de ladite seconde zone.

Comme le montre la figure unique annexée, il s'agit pour la maille dite « maille flottée », d'une construction particulière de tricot permettant d'obtenir, par exemple avec un fil synthétique plat, une surface extérieure lisse. Le motif de base définissant complètement ladite maille flottée est obtenu sur quatre rangées de mailles :
- la première rangée (1) est composée de mailles symbolisées par des « X », dont les boucles sont entièrement formées à toutes les colonnes,
- la seconde rangée (2) est composée de mailles dont les boucles sont entièrement formées aux colonnes paires (p) et dont les boucles ne sont pas du tout formées aux colonnes impaires (i), les boucles non formées étant symbolisées par un « - »,
- la troisième rangée (3) est à nouveau composée de mailles dont les boucles sont entièrement formées à toutes les colonnes et,
- la quatrième rangée (4) est composée de mailles dont les boucles sont entièrement formées aux colonnes impaires et dont les boucles ne sont pas du tout formées aux colonnes paires.

Avantageusement, l'article ou sous-article selon la présente invention est caractérisé en ce que la matière textile lisse utilisée pour la première structure est une matière textile synthétique, de préférence un fil synthétique plat, non texturé et plus préférentiellement en combinaison avec un élasthanne (fibre élastomère dotée d'une grande élasticité commercialisée notamment sous la marque Lycra).

De cette sorte, il peut être garanti que le côté qui sera en contact avec l'article de contention proprement dit permettra un glissement aisé (coefficient de frottement très faible) et donc un enfilage facilité de ce dernier sur ledit sous-article.

De préférence, l'article ou sous-article selon l'invention est caractérisé en ce que la matière textile lisse utilisée pour la première structure est un polyamide sous forme de fil.

A ce titre, tous les polyamides habituellement utilisés dans le domaine textile peuvent être employés pour la fabrication de l'article ou sous-article selon l'invention. A titre indicatif, on préférera utiliser un Nylon peu onéreux, du type Nylon 6,6 ou analogue.

Selon une autre caractéristique avantageuse, la matière textile utilisée pour la seconde structure comprend au plus 50 % en poids d'une matière textile naturelle, tel que le coton, le lin ou la soie.

Ainsi, il devient possible d'augmenter le confort global dudit article ou sous-article, notamment en augmentant l'aération du pied dans cette zone et donc en réduisant les problèmes liés à la transpiration.

Avantageusement, le matériau utilisé pour la seconde structure est une matière textile synthétique modifiée (par exemple chimiquement en greffant les groupements fonctionnels souhaités sur le squelette de base du polymère ou copolymère constituant ladite matière textile synthétique) présentant une propriété physico-chimique améliorée par rapport à la matière textile synthétique de base, telle qu'une isolation thermique, une perméabilité à la vapeur d'eau, une action bactéricide et/ou fongicide, un effet anti-odeur, etc., améliorés.

Ainsi, il devient possible d'apporter une fonctionnalité supplémentaire à l'objet de la présente invention et de proposer un produit plus ou moins personnalisé en fonction des souhaits du patient et des recommandations du médecin traitant.

Selon une autre caractéristique avantageuse, l'article ou sous-article selon l'invention est encore caractérisé en ce qu'il comporte un talon dit alternatif tricoté avec un fil synthétique plat.

Un tel talon, bien connu dans le domaine de la manufacture textile est préférentiellement tricoté avec un fil synthétique plat. Il présente notamment l'avantage d'augmenter encore le confort pour le porteur.

En alternative, et selon une autre caractéristique avantageuse, l'article ou sous-article selon l'invention est en outre caractérisé en ce qu'il comporte, au niveau du cou de pied, une zone de section plus large. On compense ainsi, de façon relativement économique, la perte de confort occasionnée par l'absence de talon alternatif.

De manière préférée, la compression ou contention exercée par l'article ou sous-article conforme à la présente invention sur le porteur au niveau de ladite première zone est inférieure à 13 hPa.

Une contention aussi faible est avant tout prévue pour faciliter l'enfilage du vêtement tout en garantissant un maintien en place suffisant et n'a pas, contrairement à la seconde zone, comme vocation première d'assurer un effet thérapeutique au niveau du pied même si un tel effet peut être éventuellement mis à profit dans certains cas.

Avantageusement, l'article ou sous-article selon l'invention est encore caractérisé en ce que la compression ou contention exercée sur le porteur au niveau de ladite seconde zone est comprise entre 13 et 30 hPa, de préférence comprise entre 20 et 30 hPa.

Cette compression plus modérée peut suffire dans des cas pathologiques moins graves dans lesquels des produits de classes plus élevées ne sont pas nécessaires.

Bien entendu, cette contention parfaitement maîtrisée pourra venir s'additionner arithmétiquement à la contention ou compression délivrée par le véritable article de contention, par exemple par un bas de contention, notamment un bas de contention de classe nettement plus élevée.

De manière plus spécifique, l'article ou sous-article selon la présente invention est caractérisé en ce qu'il s'agit d'un bas, respectivement d'un sous-bas, d'un collant, respectivement d'un sous-collant, d'un mono-collant, respectivement d'un sous-mono-collant, c'est-à-dire d'un article placé sous un collant muni d'une seule jambe, destiné à la contention d'un seul membre inférieur ou d'une chaussette, respectivement d'une sous-chaussette.

Le fonctionnement du sous-article selon l'invention est très simple et permet de faciliter grandement la mise en place sur ledit sous-article d'autres produits de contention beaucoup plus difficiles à enfiler tout seuls. En effet, il suffit de mettre en place (d'enfiler) le sous-article adapté à l'effet recherché de manière habituelle. De part sa faible contention et sa structure spécifique, aucun effort particulier n'est nécessaire à cette fin et cette opération peut être effectuée par la plus grande partie des patients. Ensuite, il suffit d'enfiler normalement le produit de contention proprement dit, ce qui est grandement facilité grâce à la surface lisse du sous-article déjà en place.

Grâce aux produits et aux procédés selon l'invention, l'application thérapeutique de produits de contention (en particulier de classe élevée ou très élevée) peut être exécutée sans effort démesuré, d'une manière précise et reproductible de sorte à minimiser les contraintes qui en découlent ainsi que leurs effets néfastes (efforts à fournir importants, erreurs de pose, suivi aléatoire voire non-respect du traitement prescrit...) et à garantir au patient un traitement médical efficace et conforme à la prescription du médecin.

La présente invention a encore pour objet un procédé de mise en place d'un article de contention, caractérisé en ce qu'il consiste essentiellement à mettre en place sur la partie du corps à traiter, préalablement à la mise en place dudit article de contention, un sous-article selon la présente invention.

De cette sorte on facilite grandement la mise en place d' articles de contention, en particulier la mise en place de ceux présentant une classe élevée et on peut apporter, si nécessaire, des fonctionnalités supplémentaires au patient.

Bien entendu, l'invention n'est pas limitée aux modes de réalisation décrits et au dessin annexé. Des modifications restent possibles, notamment du point de vue de la constitution des divers éléments ou par substitution d'équivalents techniques, sans sortir pour autant du domaine de protection de l'invention.

## Revendications

1. Article ou sous-article de contention, pouvant être utilisé seul ou préférentiellement sous un article de contention en étant en contact direct avec la peau du porteur, **caractérisé en ce qu'**il est essentiellement tricoté avec au moins deux structures de maillage différentes, à savoir :
- une première structure pour une première zone dudit article ou sous-article qui s'étend de l'extrémité prévue pour les orteils jusqu'au niveau des malléoles dudit porteur, cette zone étant tricotée selon un motif dit de « maille flottée », ce dans une matière textile présentant une surface extérieure opposée à celle en contact avec la peau dudit porteur qui soit lisse,
- une seconde structure pour une seconde zone dudit article ou sous-article qui s'étend des malléoles précitées jusqu'à l'autre extrémité opposée à celle prévue pour lesdits orteils, cette zone étant tricotée selon un motif dit de « maille tramée », ce dans une matière textile élastique adaptée présentant un titre de trame compris entre 240 dtex et 600 dtex, de façon à procurer une légère compression ou contention au niveau de ladite seconde zone.

2. Article ou sous-article selon la revendication 1, **caractérisé en ce que** la matière textile lisse utilisée pour la première structure est une matière textile synthétique, de préférence un fil synthétique plat, non texturé, et plus préférentiellement en combinaison avec un élasthanne.

3. Article ou sous-article selon la revendication 1 ou 2, **caractérisé en ce que** la matière textile lisse utilisée pour la première structure est un polyamide sous forme de fil.

4. Article ou sous-article selon l'une quelconque des revendications 1 à 3, **caractérisé en ce que** la matière textile utilisée pour la seconde structure comprend au plus 50 % en poids d'une matière textile naturelle, tel que le coton, le lin ou la soie.

5. Article ou sous-article selon l'une quelconque des revendications 1 à 4, **caractérisé en ce que** la matière textile utilisée pour la première et/ou la seconde structure comprend une matière textile synthétique modifiée présentant une propriété physico-chimique améliorée par rapport à la matière textile synthétique de base, telle qu'une isolation thermique, une perméabilité à la vapeur d'eau, une action bactéricide et/ou fongicide, un effet anti-odeur, etc., améliorés.

6. Article ou sous-article selon l'une quelconque des revendications précédentes, **caractérisé en ce qu'**il comporte un talon dit alternatif tricoté avec un fil synthétique plat.

7. Article ou sous-article selon l'une quelconque des revendications précédentes, **caractérisé en ce que** la compression ou contention exercée sur le porteur au niveau de ladite première zone est inférieure à 13 hPa.

8. Article ou sous-article selon l'une quelconque des revendications précédentes, **caractérisé en ce que** la compression ou contention exercée sur le porteur au niveau de ladite seconde zone est comprise entre 13 et 30 hPa, de préférence comprise entre 20 et 30 hPa.

9. Article ou sous-article selon l'une quelconque des revendications précédentes, **caractérisé en ce qu'**il s'agit d'un bas, respectivement d'un sous-bas.

10. Article ou sous-article selon l'une quelconque des revendications 1 à 8, **caractérisé en ce qu'**il s'agit d'un collant, respectivement d'un sous-collant.

11. Article ou sous-article selon l'une quelconque des revendications 1 à 8, **caractérisé en ce qu'**il s'agit d'un mono-collant, respectivement d'un sous-mono-collant, c'est-à-dire d'un article placé sous un collant muni d'une seule jambe, destiné à la contention d'un seul membre inférieur.

12. Article ou sous-article selon l'une quelconque des revendications 1 à 8, **caractérisé en ce qu'**il s'agit d'une chaussette, respectivement d'une sous-chaussette.

## Claims

1. Retention or compression garment or undergarment that can be used alone or, for preference, under a retention garment and in direct contact with the wearer's skin, **characterized in that** it is essentially knitted with at least two different stitch work structures, namely:
- a first structure for a first region of the said garment or undergarment which extends from the end intended for the toes as far as the ankle bones of the said wearer, this zone being knitted with a pattern known as "miss stitch" or "float stitch", and in a textile material that on the exterior surface, the surface opposite the one in contact with the skin of the said wearer, is smooth,
- the second structure for a second zone of the said garment or undergarment which extends from the aforementioned ankle bones as far as the opposite end to the end intended for the said toes, this zone being knitted with a pattern known as "weft stitch", and in a suitable elastic textile material with a weft count of between 240 dtex and 600 dtex, so as to provide slight compression or retention in the said second zone.

2. Garment or undergarment according to Claim 1, **characterized in that** the smooth textile material used for the first structure is a synthetic textile material, preferably a non-textured flat synthetic yarn, and more preferably still, one combined with a spandex.

3. Garment or undergarment according to Claim 1 or 2, **characterized in that** the smooth textile material used for the first structure is a polyamide or nylon in yarn form.

4. Garment or undergarment according to any one of Claims 1 to 3, **characterized in that** the textile material used for the second structure contains at most 50% by weight of a natural textile material such as cotton, linen or silk.

5. Garment or undergarment according to any one of Claims 1 to 4, **characterized in that** the textile material used for the first and/or the second structure comprises a modified synthetic textile material exhibiting a physico-chemical property that is improved with respect to that of the base synthetic textile material, such as improved thermal insulation, improved breathability, improved bactericidal and/or fungicidal action, and improved anti-odour effect, etc.

6. Garment or undergarment according to any one of the preceding claims, **characterized in that** it has what is known as an alternative heel knitted with a flat synthetic yarn.

7. Garment or undergarment according to any one of the preceding claims, **characterized in that** the compression or retention exerted on the wearer in the said first zone is lower than 13 hPa.

8. Garment or undergarment according to any one of the preceding claims, **characterized in that** the compression or retention exerted on the wearer in the said second zone is between 13 and 30 hPa, preferably between 20 and 30 hPa.

9. Garment or undergarment according to any one of the preceding claims, **characterized in that** it is a stocking or an underhose, respectively.

10. Garment or undergarment according to any one of Claims 1 to 8, **characterized in that** it is a pair of tights or an underhose, respectively.

11. Garment or undergarment according to any one of Claims 1 to 8, **characterized in that** it is a one-legged pair of tights or a one-legged underhose, respectively, that is to say a garment placed under a pair of tights that has just one leg, intended to retain just one lower limb.

12. Garment or undergarment according to any one of Claims 1 to 8, **characterized in that** it is a sock or an under sock, respectively.

## Patentansprüche

1. Artikel bzw. Unterziehartikel zur Kompression, der allein oder vorzugsweise unter einem Kompressionsartikel verwendet werden kann, wobei er sich in direktem Kontakt mit der Haut des Tragenden befindet, **dadurch gekennzeichnet, dass** er im Wesentlichen mit wenigstens zwei unterschiedlichen Maschenstrukturen gewirkt ist, und zwar:
- einer ersten Struktur für einen ersten Bereich des Artikels bzw. Unterziehartikels, der sich von dem für die Zehen vorgesehenen Ende bis auf Höhe der Knöchel des Tragenden erstreckt, wobei dieser Bereich in einem als "übersprungene Masche" bezeichneten Muster gewirkt ist, und zwar in einem textilen Material, das eine äußere, der sich in Kontakt mit der Haut des Tragenden gegenüberliegende Oberfläche aufweist, die glatt ist;
- einer zweiten Struktur für einen zweiten Bereich des Artikels bzw. Unterziehartikels, die sich von den genannten Knöcheln bis zu dem weiteren, dem für die Zehen vorgesehenen gegenüberliegenden Ende erstreckt, wobei dieser Bereich gemäß einem als "Schussmasche" bezeichneten Muster gewirkt ist, und zwar in einem elastischen, angepassten textilen Material, das einen Schussgarn-Titer zwischen 240 dtex und 600 dtex enthält, und derart, dass ein leichter Druck bzw. eine leichte Kompression auf Höhe des zweiten Bereichs erzeugt wird.

2. Artikel bzw. Unterziehartikel zur Kompression nach Anspruch 1, **dadurch gekennzeichnet, dass** das für die erste Struktur verwendete glatte textile Material ein synthetisches textiles Material ist, vorzugsweise ein flaches synthetisches Garn, das nicht texturiert ist, und noch vorteilhafter in Kombination mit einem Elastan.

3. Artikel bzw. Unterziehartikel zur Kompression nach Anspruch 1 oder 2, **dadurch gekennzeichnet, dass** das für die erste Struktur verwendete glatte textile Material ein Polyamid in Form eines Garns ist.

4. Artikel bzw. Unterziehartikel zur Kompression nach einem der Ansprüche 1 bis 3, **dadurch gekennzeichnet, dass** das für die zweite Struktur verwendete textile Material höchstens 50 Gewichts-% eines natürlichen textilen Materials wie etwa Baumwolle, Leinen bzw. Flachs oder Seide enthält.

5. Artikel bzw. Unterziehartikel zur Kompression nach einem der Ansprüche 1 bis 4, **dadurch gekennzeichnet, dass** das für die erste und/oder die zweite Struktur verwendete textile Material ein modifiziertes synthetisches textiles Material enthält, das eine in Bezug auf das synthetische textile Basismaterial verbesserte physikalischchemische Eigenschaft wie etwa eine verbesserte Wärmeisolierung, Durchlässigkeit gegenüber Wasserdampf, bakterizide und/oder fungizide Wirkung, geruchshemmende Wirkung etc. aufweist.

6. Artikel bzw. Unterziehartikel zur Kompression nach einem der vorgenannten Ansprüche, **dadurch gekennzeichnet, dass** er eine sogenannte mit einem flachen synthetischen Garn im Wechsel gewirkte Ferse umfasst.

7. Artikel bzw. Unterziehartikel zur Kompression nach einem der vorgenannten Ansprüche, **dadurch gekennzeichnet, dass** der Druck bzw. die Kompression, der/die auf den Tragenden in Höhe des ersten Bereichs ausgeübt wird, geringer als 13 hPa ist.

8. Artikel bzw. Unterziehartikel zur Kompression nach einem der vorgenannten Ansprüche, **dadurch gekennzeichnet, dass** der Druck bzw. die Kompression, der/die auf den Tragenden in Höhe des zweiten Bereichs ausgeübt wird, zwischen 13 und 30 hPa, vorzugsweise zwischen 20 und 30 hPa beträgt.

9. Artikel bzw. Unterziehartikel zur Kompression nach einem der vorgenannten Ansprüche, **dadurch gekennzeichnet, dass** es sich um einen Strumpf bzw. einen Unterziehstrumpf handelt.

10. Artikel bzw. Unterziehartikel zur Kompression nach einem der Ansprüche 1 bis 8, **dadurch gekennzeichnet, dass** es sich um eine Strumpfhose bzw. eine Unterziehstrumpfhose handelt.

11. Artikel bzw. Unterziehartikel zur Kompression nach einem der Ansprüche 1 bis 8, **dadurch gekennzeichnet, dass** es sich um eine Einfachstrumpfhose bzw. eine Einfach-Unterziehstrumpfhose handelt, das heißt um einen Artikel, der unter einer Strumpfhose angeordnet und mit einem einzigen Bein versehen ist und zur Kompression einer einzigen unteren Gliedmaße dient.

12. Artikel bzw. Unterziehartikel zur Kompression nach einem der Ansprüche 1 bis 8, **dadurch gekennzeichnet, dass** es sich um einen Kniestrumpf bzw. einen Unterziehkniestrumpf handelt.
